# EUROPEAN PATENT SPECIFICATION

(11) **EP 4 717 293 B1**
(45) Date of publication and mention of the grant of the patent: **25.11.2026**
(21) Application number: 25204505.9
(22) Date of filing: 25.09.2025
(51) Int. Cl.: A61M 16/10

(54) **HOME VENTILATOR**
HEIMVENTILATOR
VENTILATEUR DOMESTIQUE

(30) Priority: 28.09.2024 CN 202411364085
(43) Date of publication of application: 01.04.2026
(73) Proprietor: Hunan Taiyanglong Medical Technology Co., Ltd., Liuyang City, Hunan Province 410300 (CN)
(72) Inventor: CHEN, Lian, Liuyang City, 410300 (CN); CHEN, Zhiliang, Liuyang City, 410300 (CN); YANG, Xuwu, Liuyang City, 410300 (CN); CHEN, Sheng, Liuyang City, 410300 (CN)
(74) Representative: Chung, Hoi Kan

(56) References cited:
- CN-A- 112 169 114
- CN-U- 214 807 576
- US-A1- 2021 338 967
- US-A1- 2022 016 679
- US-A1- 2023 330 290

## Description

### TECHNICAL FIELD

The present disclosure relates to a home ventilator, and belongs to the field of home ventilators.

### BACKGROUND

Sleep apnoea syndrome has become a relatively common sleep respiratory disorder. Its main clinical manifestations are snoring during sleep and daytime sleepiness. It not only seriously affects sleep quality, but also easily leads to hypertension, diabetes, and cardiovascular and cerebrovascular diseases in a long term. In severe cases, it can even cause sudden death during sleep, making it a potentially fatal sleep respiratory disorder.

Continuous positive pressure ventilation through a home ventilator of a non-invasive ventilation structure during sleep is an effective means for treating sleep apnoea syndrome. Especially for cases caused by lung failure or obstructive sleep apnoea, the use of a ventilator can effectively improve the cases as it does not require hospital surgery or drug treatment. Its efficacy and convenience are increasingly accepted by a large number of patients. However, during the use of the home ventilator, there are still some problems and inconvenience in the storage of accessories/components of the home ventilator and use environments.

Firstly, during the use, a mouth and nose mask, a nose mask, and a nose plug arranged on the home ventilator directly make contact with a patient's face. Grease seeping from skin and bacteria and viruses produced by exhalation through the mouth and nose can accumulate and breed therein. Condensate water generated by a respiratory connecting tube and water stains in a humidification box are very likely to breed bacteria and viruses. To ensure healthy use, the home ventilator should be cleaned and disinfected in time and stored cleanly for later use after every use. However, the above conditions are not achieved in daily home storage. Therefore, even if the home ventilator is completely cleaned by a user, the above accessories/components will still be contaminated again during the storage process.

In addition, in terms of the use environments, due to the limitations of the power and structure of an air feed device, only a single layer of primary/medium-efficiency filter cotton is arranged for an air source for simple filtration. This will cause dust particles smaller than 5 µm, including bacteria and viruses in air, to be simultaneously inhaled into the home ventilator and enter the human body through airways, leading to viral infection in the lungs. Moreover, dust particles, bacteria and viruses that enter airways and various components of the home ventilator will accumulate and breed. Affected by air pollution, the air filter cotton needs to be replaced in time every half a month to a month. Due to the influence of user's personal habits, replacement time is bound to be highly uncertain and inconvenient. Objectively, there is also a certain degree of health risk. CN 214807576 U describes a low-noise portable household ventilator.

### SUMMARY

As for the above technical problems, the present disclosure provides a home ventilator. The home ventilator has a continuous positive pressure ventilation function, and meanwhile has functions of clean storage of accessories/components of the home ventilator and filtration and purification of an air source, so as to ensure that the accessories/components of the home ventilator are located in clean storage spaces, improve cleanliness of the air source required for operation, and further purify air in indoor environments.

The objectives of the present disclosure are achieved by the following technical solution:

A home ventilator including a body with a respiratory function assembly a storage device, an air purification system, and a display control component, where
the storage device is provided with a storage chamber, exhaust ports, and a storage opening capable of being opened or closed, and the storage chamber communicates with an air supply device;
the air purification system is provided with a fan, an air purification device, and air outlets; and
external air of the body is introduced from air inlets, an airflow channel in an air outflow direction of the air purification device in the body is a clean air region, and the respiratory function assembly and the air supply device respectively draw in clean air.

In this technical solution, the body is further provided with an air tube cleaning connecting portion, and the air tube cleaning connecting portion communicates with the airflow channel, and is provided with a connecting portion movable cover.

In this technical solution, the body is further provided with an air tube cleaning connecting portion, the air tube cleaning connecting portion communicates with the air supply device, the clean air drawn in by the air supply device is respectively conveyed to the storage chamber and the air tube cleaning connecting portion, and the air tube cleaning connecting portion is provided with a connecting portion movable cover.

In this technical solution, an ozone generator is arranged in an air outflow direction of the air supply device.

In this technical solution, the air supply device has a heating structure.

In this technical solution, the respiratory function assembly includes an air feed driving device, an air path channel, a respiratory tube connector, and a humidification box.

In this technical solution, the humidification box is detachably connected to the body.

In this technical solution, the storage chamber includes a fixedly or movably mounted storage and placement component, the storage and placement component is a storage plate or a storage basket formed by lattices/grids, the storage opening is sealed by an opening or closing door or a movable cover, a sealing structure is arranged between the storage opening and the opening or closing door or the movable cover, and the exhaust ports are formed in a position adaptive to the opening or closing door or the movable cover or the storage chamber.

In this technical solution, the storage opening is formed in an upper end of the storage chamber and connected and sealed by the detachable or coupled movable cover, and the storage and placement component is a storage plate.

In this technical solution, the storage opening is formed in a side portion of the storage chamber and sealed by the coupled opening or closing door, and the storage and placement component is a storage plate.

In this technical solution, the storage opening is formed in a side portion of the storage chamber, the storage and placement component is a storage basket, the storage basket and the opening or closing door form a pull structure to be movably connected to the storage chamber, and the storage opening is sealed by the opening or closing door.

In this technical solution, the air purification device is composed of one or at least two of an activated carbon filter, a cartridge filter, an anion purifier, a photocatalyst purifier, and a plasma purifier.

In this technical solution, the activated carbon filter and the cartridge filter are of a barrel-shaped structure or a platy structure.

In this technical solution, the activated carbon filter and/or the cartridge filter of the platy structure is constructed by a drawer type pull structure.

In this technical solution, the activated carbon filter and/or the cartridge filter of the barrel-shaped or platy structure is connected to the body by an embedding structure, and a movable plate is detachably connected or coupled to a portion of the body corresponding to the activated carbon filter and/or the cartridge filter.

In this technical solution, the cartridge filter is composed of one or at least two of a primary filter, a medium-efficiency filter, and a high-efficiency filter.

In this technical solution, the storage chamber is provided with at least one supporting member and/or a sterilization and disinfection device, and the supporting members bear, suspend, and fix accessories/components of the respiratory function assembly through at least one contact point or contact surface.

In this technical solution, the sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure.

In this technical solution, the display control component includes a clean storage display control component, a respiratory function display screen, and a respiratory function control key.

In this technical solution, the body is provided with movable wheels.

The present disclosure further provides a home ventilator including a body with a respiratory function assembly, a storage device, an air purification system, and a display control component, where
the storage device is provided with a storage chamber, exhaust ports, and a storage opening capable of being opened or closed, the storage chamber communicates with an air supply device, and the air supply device draws in external air of the body;
the air purification system is provided with a fan, an air purification device, and air outlets; and
the external air of the body is introduced from air inlets, an airflow channel in an air outflow direction of the air purification device in the body is a clean air region, and the respiratory function assembly draws in clean air.

The present disclosure has the following advantages and beneficial effects:
1. The body is further provided with components and spaces for storage of the accessories/components besides structural components of the home ventilator for ventilation therapy, the mouth and nose mask/nose mask/nose plug, the respiratory air tube and the humidification box can be stored therein after being used daily or cleaned and then air-dried/dried, and the clean air is used for air-drying/drying and can be continuously conveyed to the storage chamber, such that the storage chamber can be kept in a clean state, thereby meeting the requirements of clean storage of the above accessories/components stored therein and keeping sanitary safety of daily use.
2. The air purification system is further arranged in the body. Through more efficient filtration structures such as a high-efficiency filter and an activated carbon filter in the air purification system, dust particles smaller than 5 µm, as well as bacteria and viruses, are filtered and purified, thereby providing a clean air source for the air feed driving device and air-drying/drying. This not only can improve the quality of air required for ventilation and clean storage, but also can ensure the cleanliness of various components and airways in the respiratory function component, eliminating the need to clean internal structures of the air feed driving device and the air path channel. Especially for users with pulmonary failure and other conditions that require higher air quality, it also eliminates the risk of patients' lung infection caused by insufficient air filtration. The service life and replacement cycle of the air purification device are longer. No frequent replacement or maintenance is required, thereby achieving more convenient use.
3. The air feed driving device in the respiratory function assembly is mounted in an inner cavity of the body through a module, and noise of the air feed driving device is lower than that of an existing home ventilator due to double noise reduction of the module of the air feed driving device and the body, thereby providing higher-quality sleep environments for patients during use.
4. The system further has a function of purifying air in indoor environments besides non-invasive continuous positive pressure ventilation, thereby achieving multiple purposes.

### BRIEF DESCRIPTION OF DRAWINGS

FIG. 1 is a main structural diagram of Embodiment 1 of the present disclosure.
FIG. 2 is a schematic structural diagram of a direction A in FIG. 1.
FIG. 3 is a schematic structural diagram of a section cutting plane of a line B-B in FIG. 2.
FIG. 4 is a schematic structural diagram of a section cutting plane of a line C-C in FIG. 2.
FIG. 5 is a schematic diagram of an airflow flow direction in an operating state of a respiratory function assembly in Embodiment 1 of the present disclosure.
FIG. 6 is a schematic diagram of an airflow flow direction in an operating state of a respiratory function assembly in a section cutting plane of a line D-D in FIG. 5.
FIG. 7 is a schematic diagram of an airflow flow direction in an operating state for cleaning a storage chamber and an air tube in Embodiment 1 of the present disclosure.
FIG. 8 is a schematic diagram of a clean air conveying frame of the present disclosure.
FIG. 9 is a schematic structural diagram of Embodiment 2 of the present disclosure.
FIG. 10 is a schematic structural diagram of a direction E in FIG. 9.
FIG. 11 is a schematic structural diagram of a section cutting plane of a line F-F in FIG. 10.
FIG. 12 is a schematic structural diagram of a section cutting plane of a line G-G in FIG. 10.
FIG. 13 is a schematic diagram of an airflow flow direction in an operating state of a respiratory function assembly in Embodiment 2 of the present disclosure.
FIG. 14 is a schematic diagram of an airflow flow direction in an operating state of a respiratory function assembly in a section cutting plane of a line H-H in FIG. 13.
FIG. 15 is a schematic diagram of an airflow flow direction in an operating state for cleaning a storage chamber and an air tube in Embodiment 2 of the present disclosure.

Reference signs: 1-body, 2-exhaust port, 3-opening or closing door, 4-humidification box, 5-connecting portion movable cover, 6-respiratory function control key, 7-respiratory function display screen, 8-clean storage display control component, 9-respiratory tube connector, 10-air outlet, 11-storage chamber, 12-fan, 13-air purification device, 14-air inlet, 15-airflow channel, 16-ozone generator, 17-air tube, 18-air tube cleaning connecting portion, 19-air outflow tube, 20-air feed driving device, 21-main control module, 22-air inflow portion, 23-storage and placement component, 24-air supply device, 25-air chamber, 26-respiratory air tube, 27-air feed tube, 28-respiratory mask, and 29-movable cover.

### DETAILED DESCRIPTION OF THE EMBODIMENTS

The present disclosure will be described in detail below with reference to accompanying drawings and specific embodiments.

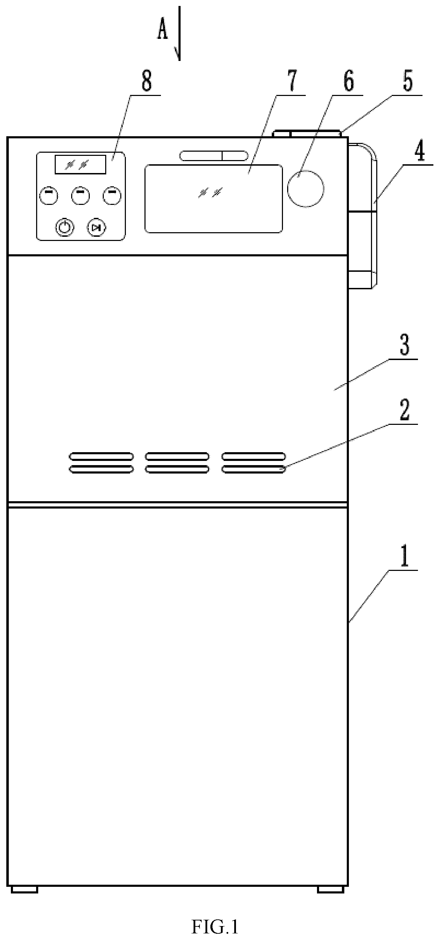

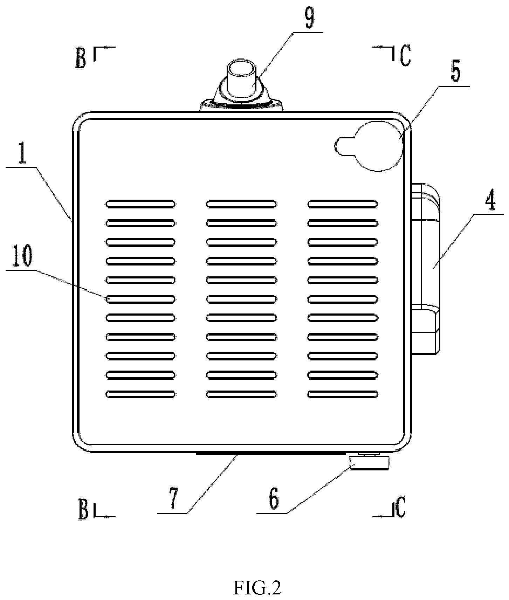

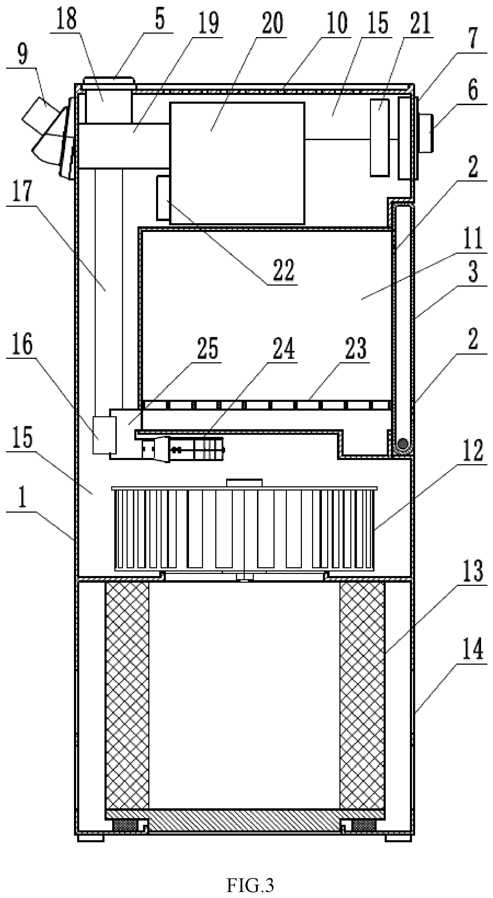

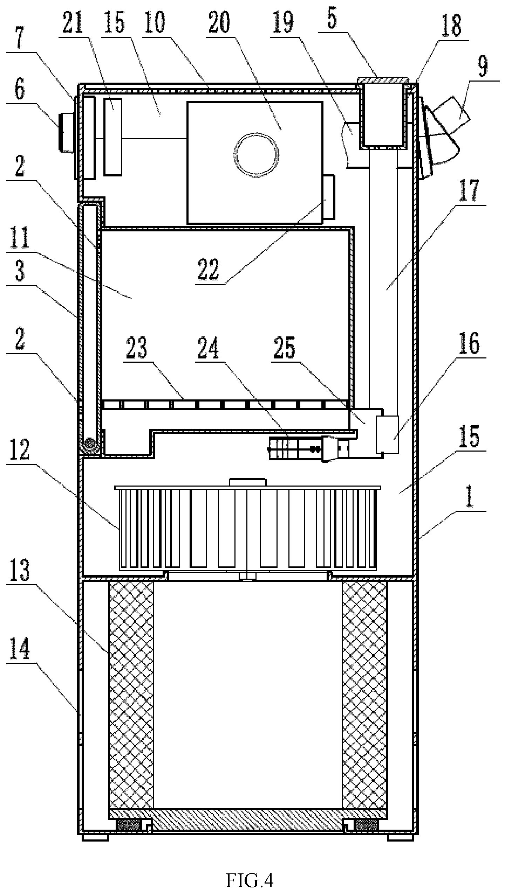

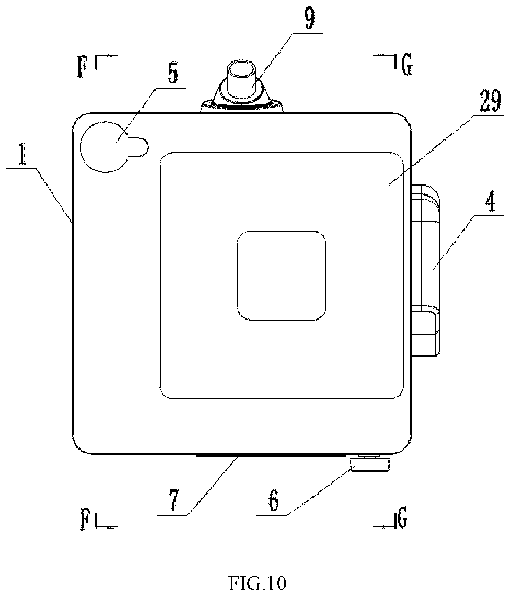

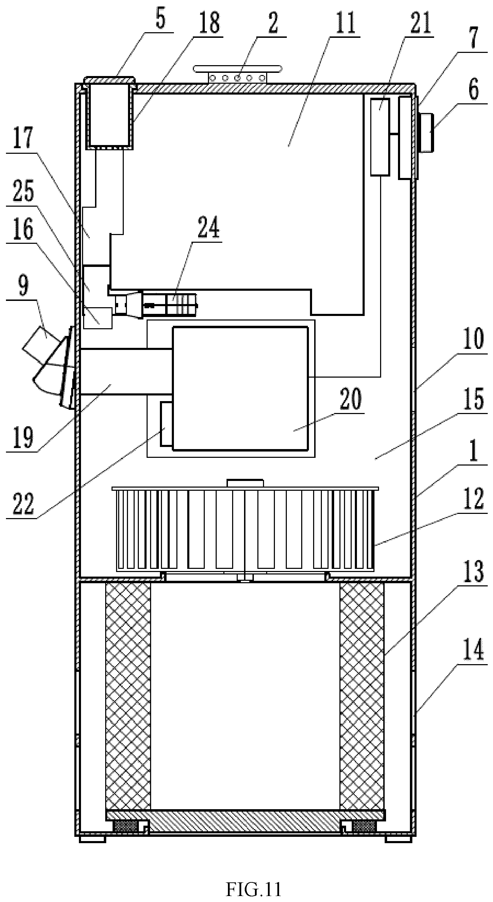

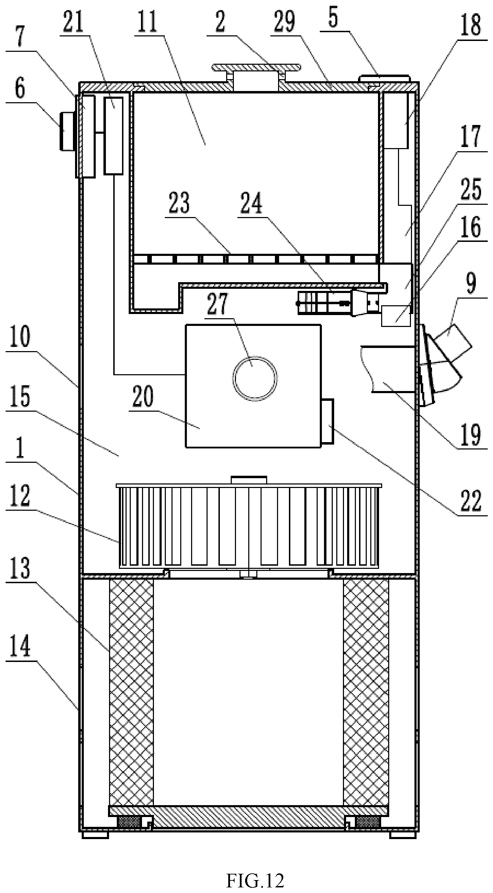

FIG. 1, FIG. 2, FIG. 3, FIG. 4, FIG. 5, FIG. 6, FIG. 7, FIG. 8, FIG. 9, FIG. 10, FIG. 11, FIG. 12, FIG. 13, FIG. 14, and FIG. 15 illustrate embodiments of the present disclosure. A home ventilator is formed by assembling a body 1, exhaust ports 2, an opening or closing door 3, a humidification box 4, a connecting portion movable cover 5, a respiratory function control key 6, a respiratory function display screen 7, a clean storage display control component 8, a respiratory tube connector 9, air outlets 10, a storage chamber 11, a fan 12, an air purification device 13, air inlets 14, an airflow channel 15, an ozone generator 16, an air tube 17, an air tube cleaning connecting portion 18, an air outflow tube 19, an air feed driving device 20, a main control module 21, an air inflow portion 22, a storage and placement component 23, an air supply device 24, an air chamber 25, a respiratory air tube 26, an air feed tube 27, a respiratory mask 28, a movable cover 29, connecting components, and fasteners.

Specific embodiments have the following structural characteristics.

A home ventilator including a body 1 with a respiratory function assembly, a storage device, an air purification system, and a display control component, where
the storage device is provided with a storage chamber 11, exhaust ports 2, and a storage opening capable of being opened or closed, and the storage chamber 11 communicates with an air supply device 24;
the air purification system is provided with a fan 12, an air purification device 13, and air outlets 10; and
external air of the body is introduced from air inlets 14, an airflow channel 15 in an air outflow direction of the air purification device in the body is a clean air region, and the respiratory function assembly and the air supply device 24 respectively draw in clean air.

In the embodiment of the present disclosure, the respiratory function assembly includes an air feed driving device 20, an air path channel, a respiratory tube connector 9, and a humidification box 4, all of which are respectively mounted on the body 1. The respiratory function assembly may be mounted on an adaptive portion of the body 1, such as an upper portion, a middle portion or a lower portion of the body 1. The storage chamber 11 is used for storing, air-drying/drying accessories/components of the respiratory function assembly. The accessories/components include, but are not limited to a respiratory mask 28, a nose mask, a nose plug, a respiratory air tube 26, and the humidification box 4.

In some embodiments, the humidification box 4 may be fixedly mounted on the body 1, or detachably mounted on the body 1 according to actual use requirements, and the humidification box 4 is convenient to detach and clean due to a detachable mounting structure.

In the embodiment of the present disclosure, the air supply device 24 is provided with an air feed driving component and air inlets, and an air outflow direction of the air feed driving component communicates with the storage chamber 11.

In the embodiment of the present disclosure, the external air is introduced into the body 1 through the air inlets 14 via negative pressure produced by the fan 12, and filtered and purified by the air purification device 13 into the clean air. A clean air flow channel is formed by the airflow channel 15 in the air outflow direction of the air purification device 13 in the body 1. An air inflow portion 22 of the air feed driving device 20 draws in the clean air as an air source required for ventilation. An air intake vent of the air supply device 24 draws in the clean air and conveys the same to the storage chamber 11, the clean air in the storage chamber 11 is exhausted from the exhaust ports 2, and meanwhile the clean air is further conveyed to an indoor environment through the air outlets 10. Thus, the body 1 not only has a ventilation therapy function, provides the clean air for the respiratory function assembly to serve as the air source required for ventilation therapy, improves cleanliness of the air source for ventilation therapy, and ensures cleanliness of the components in the respiratory function assembly and the air path channel, but also can provide a clean storage space environment for the accessories/components of the respiratory function assembly, air-dry water stains left after cleaning the accessories/components of the respiratory function assembly, and purify environment air.

In some embodiments, the air supply device 24 may communicate with any applicable position such as a bottom, a side face or an upper portion of the storage chamber 11.

In some embodiments, the body 1 may be provided with an air tube cleaning connecting portion 18, the air tube cleaning connecting portion communicates with the airflow channel 15 for the introduction of the clean air, the respiratory air tube 26 can be connected to the air tube cleaning connecting portion after being used or cleaned, so as to be air-dried/dried or only suspended. The air tube cleaning connecting portion is provided with a connecting portion movable cover 5 with a protection effect in a non-use state.

In some embodiments, for example, the body 1 is provided with the air tube cleaning connecting portion 18 and the connecting portion movable cover 5, and the air tube cleaning connecting portion 18 communicates with the air supply device 24, such that the clean air drawn in by the air supply device 24 is respectively conveyed to the storage chamber 11 and the air tube cleaning connecting portion 18, and the air supply device 24 conveys the clean air to the air tube cleaning connecting portion 18 while conveying the clean air to the storage chamber 11.

In some embodiments, the air outflow direction of the air supply device 24 may be provided with an ozone generator 16, such that the air supply device 24 can convey ozone to the storage chamber 11 for zone sterilization and disinfection on the storage chamber 11 and the accessories/components of the respiratory function assembly stored in the storage chamber. Moreover, in the embodiment where the air supply device communicates with the air tube cleaning connecting portion 18, the air supply device 24 can further convey the ozone to the respiratory air tube 26 connected with the air tube cleaning connecting portion 18 for ozone sterilization and disinfection on the respiratory air tube.

In some embodiments, the air supply device 24 may have a heating structure, and the heating structure can heat the clean air drawn in by the air supply device 24 and then convey the same to the storage chamber 11, such that the accessories/components of the respiratory function assembly stored in the storage chamber 11 can be dried and sterilized and disinfected at a high temperature. In the embodiment where the air supply device communicates with the air tube cleaning connecting portion 18, the air supply device 24 can further convey the heated clean air to the respiratory air tube 26 connected with the air tube cleaning connecting portion 18 for drying and ozone sterilization and disinfection on the respiratory air tube.

In the embodiment of the present disclosure, the storage chamber 11 is provided with a storage and placement component 23 and the storage opening, the storage and placement component 23 is a storage plate or a storage basket formed by lattices/grids, the storage and placement component 23 is fixedly and movably mounted in the storage chamber 11, and the storage opening can be sealed by a movable cover 29 or an opening or closing door 3 according to different arrangement portions of the storage opening of the storage chamber 11.

In the embodiment of the present disclosure, the exhaust ports 2 of the storage chamber 11 may be formed in any adaptive positions, or may be formed in the opening or closing door 3 or the movable cover 29.

In the embodiment of the present disclosure, a sealing structure is arranged on a contact portion of the movable cover 29 or the opening or closing door 3 and the storage opening of the storage chamber 11, which may be a staggered sealing structure or a rubber sealing ring.

In some embodiments, in a case where the storage opening is formed in an upper end of the storage chamber 11, the storage opening may be sealed through the movable cover 29 or the opening or closing door 3.

In some embodiments, in a case where the storage opening is formed in a side portion of the storage chamber 11, the storage opening may be sealed through the opening or closing door 3.

In the embodiment of the present disclosure, the movable cover 29 is coupled or detachably connected to the storage chamber 11, and the opening or closing door 3 is coupled to the storage chamber 11.

In some embodiments, for example, the storage and placement component 23 is a storage basket and is connected with the opening or closing door 3 to form a pull basket structure to be movably connected to the storage chamber 11.

In the embodiment of the present disclosure, the storage chamber 11 may be provided with at least one supporting member, which may be arranged at a lower end, an upper end or a side end of the storage chamber 11, or may be arranged on the storage and placement component 23. The supporting members bear, suspend, and fix the accessories/components of the respiratory function assembly through at least one contact point or contact surface. The contact points or contact surfaces adaptive to structures of the accessories/components are formed by the arrangement number and structures of the supporting elements, for example, in a case where arc-shaped structures are arranged in the accessories/components, the contact surfaces therebetween can be adaptive through the supporting elements of the arc-shaped structures, or contact points therebetween are adaptive through two or more supporting elements of columnar structures in a multi-point contact manner, for example, the supporting elements are arranged to be in a rod shape or a hook shape, such that the accessories can be stably borne, suspended and fixed.

In the embodiment of the present disclosure, the air purification device 13 is composed of one or at least two of an activated carbon filter, a cartridge filter, an anion purifier, a photocatalyst purifier, and a plasma purifier.

That is, the air purification device 13 may be composed of one of the above structures, or may be composed of two or more of the above structures. In a case where two air purification structures are adopted, air is sequentially filtered and purified through the two different purification structures, so as to achieve a better air purification effect.

For example, in some embodiments, two air purification devices 13 are arranged, namely the activated carbon filter and the cartridge filter. Air can be purified by the cartridge filter and then secondarily purified by the activated carbon filter, or the air is purified by the activated carbon filter and then secondarily purified by the cartridge filter. A sequence of the two air purification structures can be determined as required according to positions where they are mounted on an airflow path. The same is true for the embodiments adopting other purifier structures.

In some embodiments, the cartridge filter is composed of one or a combination of at least two of a primary filter, a medium-efficiency filter, and a high-efficiency filter. That is, the cartridge filter may be composed of one of the above structures, or may be composed of a combination of two or more of the above structures.

For example, in some embodiments, the primary filter, the medium-efficiency filter, and the high-efficiency filter can be combined to form the cartridge filter.

For example, in some other embodiments, in a case where two air purification devices 13 are arranged, one is a cartridge filter composed of the primary filter, and the other is a cartridge filter composed of the high-efficiency filter.

In some embodiments, the air purification device 13 is, for example, embedded into the body 1 through a barrel-shaped or platy structure, and a detachably connected or coupled movable plate may be mounted on such portion of the body 1, such that the air purification device 13 can be conveniently maintained and replaced.

In some embodiments, the storage chamber 11 may be provided with a sterilization and disinfection device. The sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure. The storage chamber 11 and the accessories/components stored therein can be sterilized and disinfected through the above sterilization and disinfection device, thereby further improving the cleanliness of a space environment of the storage chamber 11 and the accessories/components stored therein.

For example, in some embodiments, the storage chamber 11 is provided with one sterilization and disinfection device, which is the UV ultraviolet sterilization and disinfection structure. In some other embodiments, the storage chamber 11 is provided with two sterilization and disinfection devices, namely the UV ultraviolet sterilization and disinfection structure and the plasma sterilization and disinfection structure. In the embodiment where the two sterilization and disinfection devices are mounted, two identical sterilization and disinfection structures may be adopted, for example, the both the two sterilization and disinfection devices are of the UV ultraviolet sterilization and disinfection structure.

In the embodiment of the present disclosure, the body 1 is provided with the display control component including a respiratory function display screen 7 and a respiratory function control key 6 for displaying and controlling a respiratory function, a clean storage display control component 8 for a clean storage function, and the above functional components are connected and controlled by a main control module 21.

In these embodiments where the sterilization and disinfection device is arranged, when the respiratory mask 28, the humidification box 4, and the respiratory air tube 26 are cleaned and then placed in the storage chamber 11, corresponding keys in the clean storage display control component 8 and the main control module control regular start and stop of the fan 12, the air supply device 24, the air purification device 13, and the sterilization and disinfection device, the clean air or heated cleaned air is intermittently or continuously conveyed to the storage chamber 11 and a disinfection medium of the sterilization and disinfection device to perform clean storage on the respiratory mask 28, the humidification box 4, and the respiratory air tube 26.

In some embodiments, the body 1 may be provided with movable wheels, such that the home ventilator can be conveniently moved to different positions for use.

FIG. 1, FIG. 2, FIG. 3, FIG. 4, FIG. 5, FIG. 6, FIG. 7, and FIG. 8 illustrate Embodiment 1 of the present disclosure.

In Embodiment 1, an air purification device 13 and a fan 12 are sequentially arranged on a lower portion of an inner cavity of a body 1 from bottom to top, the air purification device 13 is of a barrel-shaped structure, and a plurality of air inlets 14 are formed in a portion, on the air purification device 13, of the body 1.

A storage chamber 11 is arranged above the fan 12, a storage opening is formed in a side portion of the storage chamber 11, the storage opening is opened or closed through a coupled opening or closing door 3, an airflow cavity is formed in the opening or closing door 3, exhaust ports 2 are respectively formed in an inner side face and an outer side face of the opening or closing door 3, a storage and placement component 23 formed by a storage plate is mounted on a lower portion of the storage chamber 11, and an air outflow direction of an air supply device 24 communicates with a lower end of the storage chamber 11 through an air chamber 25.

An air feed driving device 20, a respiratory tube connector 9, and a humidification box 4 in the respiratory function assembly are respectively mounted at a position, above the storage chamber 11, of an upper portion of the inner cavity of the body 1, the humidification box 4 is detachably connected to the body 1, the air feed driving device 20 is connected with the humidification box 4 through an air feed tube 27, the humidification box 4 is connected with the respiratory tube connector 9 through an air outflow tube 19, and the air feed driving device 20 communicates with the humidification box 4 and the respiratory tube connector 9 to form an air path channel.

An air tube cleaning connecting portion 18 with a connecting portion movable cover 5 is mounted at an upper end of the body 1, and the air tube cleaning connecting portion 18 is connected with the air chamber 25 through an air tube 17.

The air supply device 24 has a heating structure, and the air chamber 25 is provided with an ozone generator 16.

An air outflow direction of the air purification device 13 is an airflow channel 15 for clean air to flow, and an air inflow portion 22 of the air feed driving device 20 and an air intake vent of the air supply device 24 are respectively located in the airflow channel 15, such that the air feed driving device 20 and the air supply device 24 both draw in the clean air.

The upper portion of the body 1 is respectively provided with a clean storage display control component 8, a respiratory function display screen 7, a respiratory function control key 6, and a main control module 21, and the main control module 21 is respectively connected with the clean storage display control component 8, the respiratory function display screen 7, the respiratory function control key 6, the fan 12, the air supply device 24, the ozone generator 16, and the air feed driving device 20, and receives, processes and sends corresponding information to control the above functional components to work.

A plurality of air outlets 10 communicating with the airflow channel 15 are formed in a top end of the body 1.

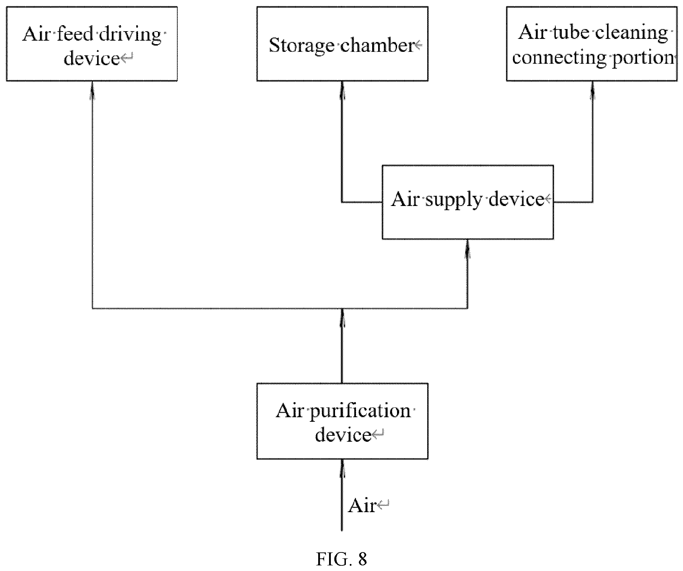

FIG. 8 is a schematic diagram of a clean air conveying frame of Embodiment 1. Clean air filtered and purified by the air purification device 13 is respectively conveyed to the air feed driving device 20 and the air supply device 24, and after being heated by the air supply device 24, the clean air is respectively conveyed to the storage chamber 11 and the air tube cleaning connecting portion 18.

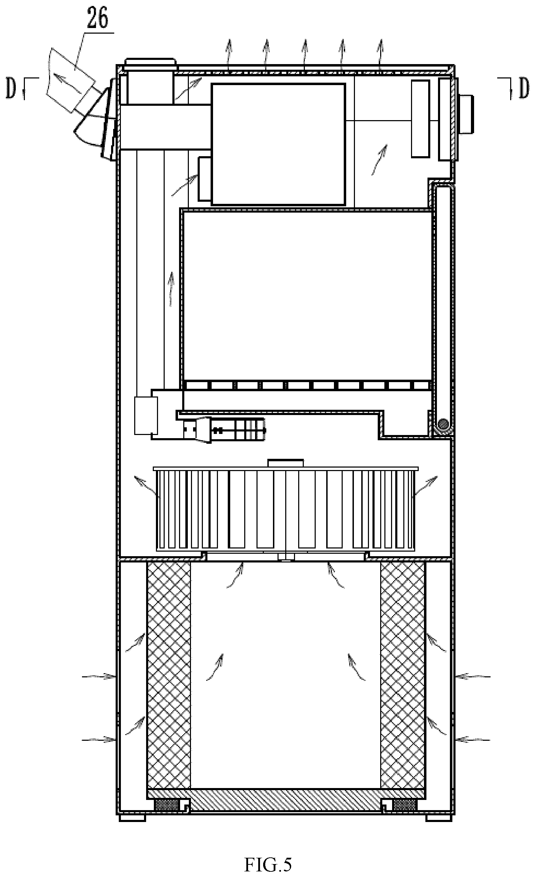

FIG. 5, FIG. 6, and FIG. 7 illustrate an airflow flow direction in an operating state of Embodiment 1.

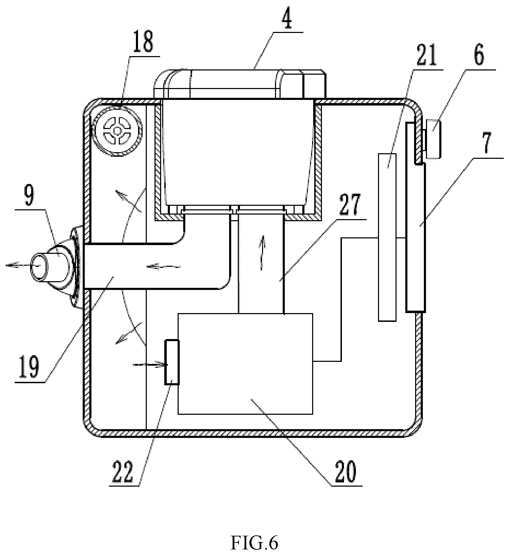

Referring to FIG. 5 and FIG. 6, FIG. 5 and FIG. 6 illustrate an airflow flow direction in an operating state of the respiratory function assembly in Embodiment 1. When a patient uses the respiratory function assembly for ventilation therapy, air enters the inner cavity of the body 1 from the air inlets 14 via negative pressure airflow produced by starting the fan 12, after the air is filtered and purified by the air purification device 13, the airflow channel 15 for the clean air to flow is formed in the inner cavity of the body 1 in an air outflow direction of the fan 12, the clean air enters the airflow channel through the air inflow portion 22 of the air feed driving device 20, and is conveyed into the humidification box 4 through the air feed tube 27 and conveyed to the respiratory tube connector 9 through the air outflow tube 19, the patient can receive ventilation therapy by connecting the respiratory air tube 26 with the respiratory tube connector 9 and wearing the respiratory mask 28 connected with the other end of the respiratory air tube, and the clean air is conveyed to the indoor environment through the air outlets 10.

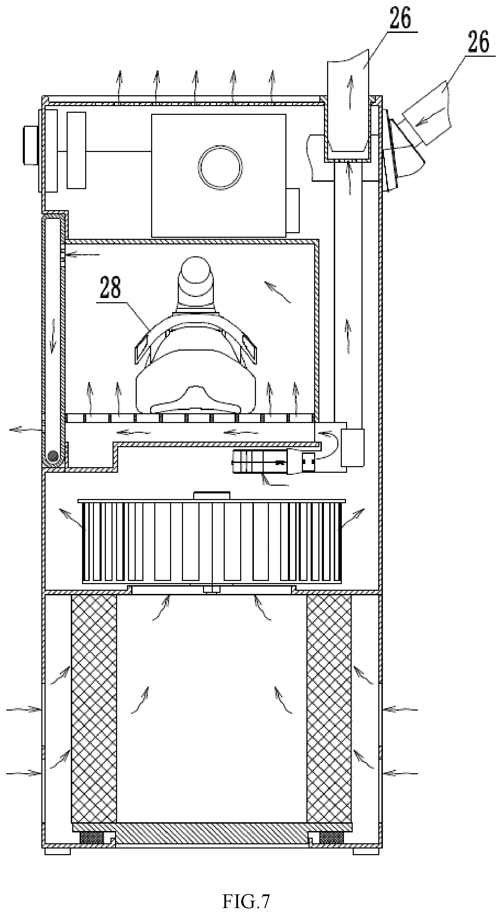

Referring to FIG. 7, FIG. 7 is an airflow flow direction in an operating state for drying the respiratory mask 28 and the respiratory air tube 26 in Embodiment 1. After the patient self-cleans and places the respiratory mask 28 in the storage chamber 11, air enters the inner cavity of the body 1 from the air inlets 14 through negative pressure airflow produced by starting the fan 12, after the air is filtered and purified by the air purification device 13, the airflow channel 15 for the clean air to flow is formed in the inner cavity of the body 1 in an air outflow direction of the fan 12, the air supply device 24 is started, the clean air is introduced from the airflow channel 15 via the air intake vent, the clean air is heated by the air supply device 24, the heated clean air is conveyed to the storage chamber through the air chamber 25 to dry the respiratory mask 28, and the clean air and water vapor in the storage chamber 11 are exhausted from the exhaust ports 2 in the opening or closing door 3.

When the respiratory air tube 26 is dried, the connecting portion movable cover 5 is opened, one end of the respiratory air tube 26 is inserted into the air tube cleaning connecting portion 18, and the other end of the respiratory air tube is connected with the respiratory tube connector 9. In a case where the humidification box 4 is detached, the air supply device 24 is started, and the clean air heated by the air supply device 24 is conveyed to the respiratory air tube 26 through the air tube 17 and the air tube cleaning connecting portion 18, and meanwhile exhausted out of the body 1 through the respiratory tube connector 9 and the air outflow tube 19. During this process, the other end of the respiratory air tube 26 may not be connected with the respiratory tube connector 9, and at this time, the heated clean air for drying the respiratory air tube is exhausted from the other end of the respiratory air tube.

It is to be noted that when the heated clean air is conveyed to the storage chamber 11 and the air tube cleaning connecting portion 18, the ozone generator 16 arranged on the air chamber 25 may be started as required, which can perform ozone sterilization and disinfection on the respiratory air tube 26, the storage chamber 11 and the accessories/components stored in the storage chamber, the ozone generator 16 may be independently used in a non-drying state as required, and it is only necessary to control start and stop of the heating structure of the air supply device 24.

After the respiratory mask 28 and the respiratory air tube 26 are dried, the main control module 21 regularly controls start and stop of the fan 12 and the air supply device 24, the heated clean air is intermittently or continuously conveyed to the storage chamber 11 and the air tube cleaning connecting portion 18, so as to achieve the clean storage function of the accessories/components of the respiratory function assembly such as the respiratory mask 28 or the respiratory air tube 26.

It is to be noted that the heating structure of the air supply device 24 can heat the clean air to a required suitable temperature, and the air supply device 24 can output the heated clean air at a certain temperature accordingly, so as to meet the requirements of drying, high-temperature sterilization and disinfection, and clean storage.

It is to be noted that the air supply device 24 and the heating structure of the air supply device 24 are both relevant high-quality products available on the market. As the prior art, their specific structures are not repeated here.

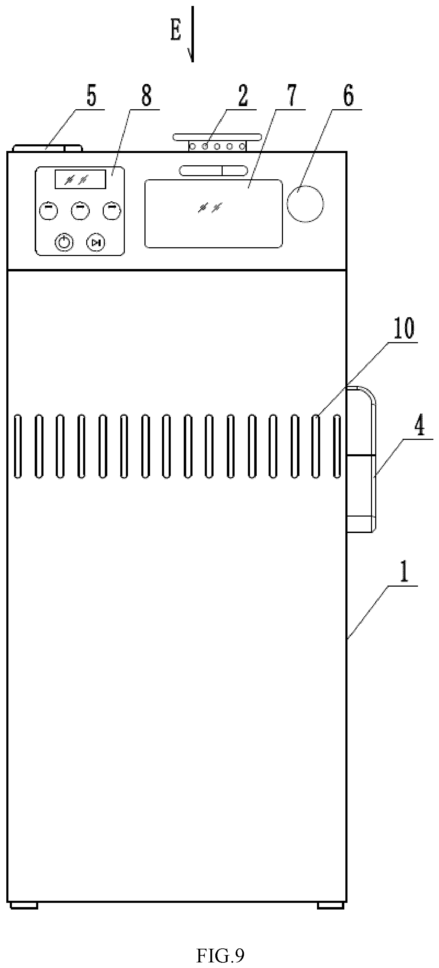

FIG. 9, FIG. 10, FIG. 11, FIG. 12, FIG. 13, FIG. 14, and FIG. 15 illustrate Embodiment 2 of the present disclosure.

In Embodiment 2, an air purification device 13 and a fan 12 are sequentially arranged on a lower portion of an inner cavity of a body 1 from bottom to top, the air purification device 13 is of a barrel-shaped structure, and a plurality of air inlets 14 are formed in a portion, on the air purification device 13, of the body 1.

An air feed driving device 20, a respiratory tube connector 9, and a humidification box 4 in a respiratory function assembly are respectively mounted above the fan 12, the humidification box 4 is detachably connected to the body 1, the air feed driving device 20 is connected with the humidification box 4 through an air feed tube 27, the humidification box 4 is connected with the respiratory tube connector 9 through an air outflow tube 19, and the air feed driving device 20 communicates with the humidification box 4 and the respiratory tube connector 9 to form an air path channel.

A storage chamber 11 is arranged on an upper portion of the inner cavity of the body 1, a storage opening is formed in an upper end of the storage chamber 11, the storage opening is sealed by a detachable movable cover 29, exhaust ports 2 are formed in the movable cover 29, a storage and placement component 23 formed by a storage plate is mounted on a lower portion of the storage chamber 11, and the air supply device 24 communicates with a lower end of the storage chamber 11 through an air chamber 25.

An air tube cleaning connecting portion 18 with a connecting portion movable cover 5 is mounted at an upper end of the body 1, and the air tube cleaning connecting portion 18 is connected with the air chamber 25 through an air tube 17.

The air supply device 24 has a heating structure, and the air chamber 25 is provided with an ozone generator 16.

An air outflow direction of the air purification device 13 is an airflow channel 15 for clean air to flow, and an air inflow portion 22 of the air feed driving device 20 and an air intake vent of the air supply device 24 are respectively located in the airflow channel 15, such that the air feed driving device 20 and the air supply device 24 both draw in the clean air.

The upper portion of the body 1 is respectively provided with a clean storage display control component 8, a respiratory function display screen 7, a respiratory function control key 6, and a main control module 21, and the main control module 21 is respectively connected with the clean storage display control component 8, the respiratory function display screen 7, the respiratory function control key 6, the fan 12, the air supply device 24, the ozone generator 16, and the air feed driving device 20, and receives, processes and sends corresponding information to control the above functional components to work.

A plurality of air outlets 10 communicating with the airflow channel 15 are formed in a middle portion of the body 1.

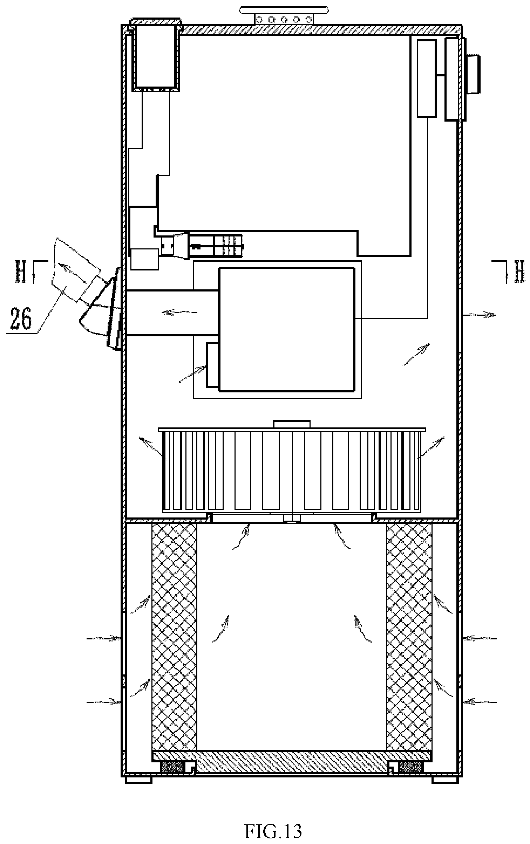

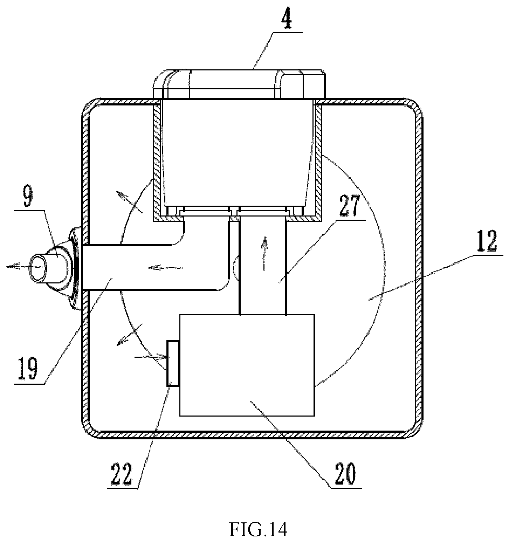

FIG. 13 and FIG. 14 illustrate an airflow flow direction in Embodiment 2 where a patient uses the respiratory function assembly for ventilation therapy. Although mounting positions of the air feed driving device 20 and the air supply device 24 change, the clean air is drawn in from the airflow channel 15, and except for the condition that the clean air is conveyed to an indoor environment through the air outlets 10 in the middle portion of the body 1, the other flow directions are the same as those in Embodiment 1, which are not repeated here.

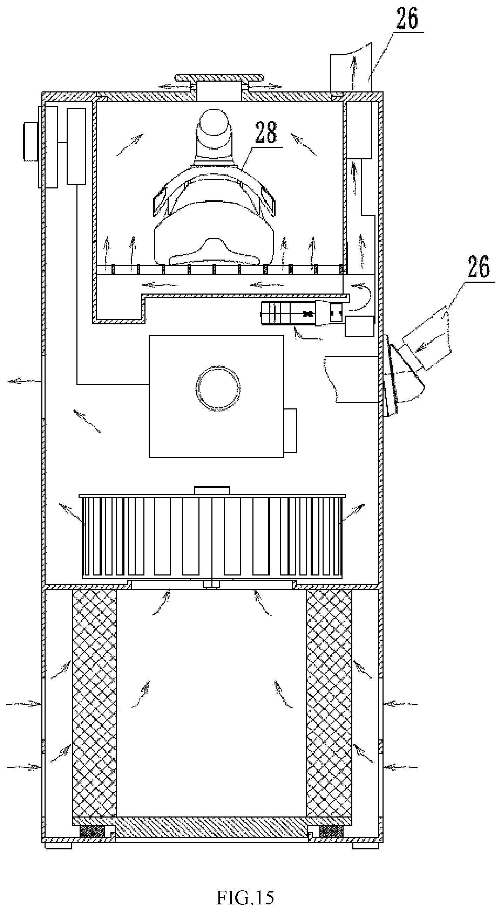

FIG. 15 illustrates an airflow flow direction in an operating state for drying a respiratory mask 28 and a respiratory air tube 26 in Embodiment 2. Except for the condition that water vapor in the storage chamber 11 is exhausted from the exhaust ports 2 in an upper end of the movable cover 29, the rest is the same as that in Embodiment 1, which is not repeated here.

In some embodiments, an air intake vent of the air supply device 24 communicates with an exterior of the body 1, air outside the body 1 is directly drawn in by the air supply device to serve as an air source for air-drying/drying, and other component settings and specific structures may adopt the contents in Embodiment 1 and Embodiment 2.

It is to be noted that in the embodiment of the present disclosure, how to mount, connect, and arrange the air feed driving device, the air path channel, and the humidification box to form a double-horizontal/single-horizontal non-invasive continuous positive pressure ventilation and humidification structure is the prior art, which is not repeated here. The above components are relevant high-quality products available on the market, which should be known to those skilled in the art.

It is to be noted that the UV ultraviolet sterilization and disinfection structure, the plasma sterilization and disinfection structure, and the photocatalyst sterilization and disinfection structure are all the prior general art and are relevant high-quality products available on the market, which should be known to those skilled in the art, and their specific connecting structures are not repeated here.

It is to be noted that a specific structure and connecting and mounting manners of the air purification device 13 are not exhaustive in the embodiment of the present disclosure. For example, an air purification device of a plate-shaped structure can be applied to the embodiment of the present disclosure as long as it is an air purification structure used for relevant high-quality products available on the market or existing air purifiers.

The activated carbon filter, the cartridge filter, the anion purifier, the photocatalyst purifier, the plasma purifier, the primary filter, the medium-efficiency filter, and the high-efficiency filter are all the prior art and are relevant high-quality products available on the market, and their specific connecting structures are not repeated here, which should be known to those skilled in the art.

As for various air purification structures adopted by the air purification device 13 in the embodiment of the present disclosure, as existing mature arts, whether they can filter particulate matter, bacteria, allergens, etc., filtration and purification effects they can achieve are all reflected in test reports and product introductions of various products available on the market. Therefore, compared with the prior art, the clean air filtered and purified by the air purification device 13 as the air source used for the respiratory function assembly, drying, high-temperature disinfection, and clean storage is significantly improved.

It is to be noted that in the embodiment of the present disclosure, better purposes of drying and sterilization and disinfection can be achieved by controlling a certain temperature of the heating structure of the air supply device 24, and please refer to pasteurism and dry heat sterilization for sterilization and disinfection principles and required temperatures.

It is to be understood that although the present disclosure mainly aims to solve the problems about daily home use scenes, it does not mean that the home ventilator cannot be used by a user in other places (such as medical care, rehabilitation, health care and other places with needs) after purchase.

The present invention is defined by the appended claims.

## Claims

1. A home ventilator comprising a body (1) with a respiratory function assembly, a storage device, an air purification system, and a display control component, wherein
the storage device is provided with a storage chamber (11), exhaust ports (2), and a storage opening capable of being opened or closed, and the storage chamber communicates with an air supply device (24);
the air purification system is provided with a fan (12), an air purification device (13), and air outlets (10); and
external air of the body is introduced from air inlets (14), an airflow channel (15) in an air outflow direction of the air purification device in the body is a clean air region, and the respiratory function assembly and the air supply device respectively draw in clean air.

2. The home ventilator according to claim 1, **characterized in that** the body (1) is further provided with an air tube cleaning connecting portion (18), and the air tube cleaning connecting portion communicates with the airflow channel (15), and is provided with a connecting portion movable cover (5).

3. The home ventilator according to claim 1, **characterized in that** the body (1) is further provided with an air tube cleaning connecting portion (18), the air tube cleaning connecting portion communicates with the air supply device (24), the clean air drawn in by the air supply device (24) is respectively conveyed to the storage chamber (11) and the air tube cleaning connecting portion, and the air tube cleaning connecting portion is provided with a connecting portion movable cover (5).

4. The home ventilator according to claim 1 or 3, **characterized in that** an ozone generator (16) is arranged in an air outflow direction of the air supply device (24).

5. The home ventilator according to claim 1 or 3, **characterized in that** the air supply device (24) has a heating structure.

6. The home ventilator according to claim 1, **characterized in that** the respiratory function assembly comprises an air feed driving device (20), an air path channel, a respiratory tube connector (9), and a humidification box (4).

7. The home ventilator according to claim 6, **characterized in that** the humidification box (4) is detachably connected to the body (1).

8. The home ventilator according to claim 1, **characterized in that** the storage chamber (11) comprises a fixedly or movably mounted storage and placement component (23), the storage and placement component is a storage plate or a storage basket formed by lattices/grids, the storage opening is sealed by an opening or closing door (3) or a movable cover (29), a sealing structure is arranged between the storage opening and the opening or closing door or the movable cover, and the exhaust ports (2) are formed in a position adaptive to the opening or closing door or the movable cover or the storage chamber.

9. The home ventilator according to claim 8, **characterized in that** the storage opening is formed in an upper end of the storage chamber (11) and connected and sealed by the detachable or coupled movable cover (29), and the storage and placement component (23) is a storage plate.

10. The home ventilator according to claim 8, **characterized in that** the storage opening is formed in a side portion of the storage chamber (11) and sealed by the coupled opening or closing door (3), and the storage and placement component (23) is a storage plate.

11. The home ventilator according to claim 8, **characterized in that** the storage opening is formed in a side portion of the storage chamber (11), the storage and placement component (23) is a storage basket, the storage basket and the opening or closing door (3) form a pull structure to be movably connected to the storage chamber, and the storage opening is sealed by the opening or closing door.

12. The home ventilator according to claim 1, **characterized in that** the air purification device (13) is composed of one or at least two of an activated carbon filter, a cartridge filter, an anion purifier, a photocatalyst purifier, and a plasma purifier.

13. The home ventilator according to claim 12, **characterized in that** the activated carbon filter and the cartridge filter are of a barrel-shaped structure or a platy structure.

14. The home ventilator according to claim 13, **characterized in that** the activated carbon filter and/or the cartridge filter of the barrel-shaped or platy structure is connected to the body (1) by an embedding structure, and a movable plate is detachably connected or coupled to a portion of the body (1) corresponding to the activated carbon filter and/or the cartridge filter.

15. The home ventilator according to claim 13, **characterized in that** the activated carbon filter and/or the cartridge filter of the platy structure is constructed by a drawer type pull structure.

16. The home ventilator according to any one of claims 12, 13, 14, and 15, **characterized in that** the cartridge filter is composed of one or at least two of a primary filter, a medium-efficiency filter, and a high-efficiency filter.

17. The home ventilator according to any one of claims 1, 3, 8, 9, 10, and 11, **characterized in that** the storage chamber (11) is provided with at least one supporting member and/or a sterilization and disinfection device, and the supporting members bear, suspend, and fix accessories/components of the respiratory function assembly through at least one contact point or contact surface.

18. The home ventilator according to claim 17, **characterized in that** the sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure.

19. The home ventilator according to claim 1, **characterized in that** the display control component comprises a clean storage display control component (8), a respiratory function display screen (7), and a respiratory function control key (6).

20. The home ventilator according to any one of claims 1, 2, 3, 7, and 14, **characterized in that** the body (1) is provided with movable wheels.

21. A home ventilator comprising a body (1) with a respiratory function assembly, a storage device, an air purification system, and a display control component, wherein
the storage device is provided with a storage chamber (11), exhaust ports (2), and a storage opening capable of being opened or closed, the storage chamber communicates with an air supply device (24), and the air supply device draws in external air of the body;
the air purification system is provided with a fan (12), an air purification device (13), and air outlets (10); and
the external air of the body is introduced from air inlets (14), an airflow channel (15) in an air outflow direction of the air purification device in the body is a clean air region, and the respiratory function assembly draws in clean air.

## Patentansprüche

1. Heimbeatmungsgerät, umfassend einen Gehäusekörper (1) mit einer Atemfunktionsbaugruppe, einer Aufbewahrungsvorrichtung, einem Luftreinigungssystem und einer Anzeigesteuerkomponente, wobei
die Aufbewahrungsvorrichtung mit einer Aufbewahrungskammer (11), Auslassöffnungen (2) und einer Aufbewahrungsöffnung, die geöffnet oder geschlossen werden kann, versehen ist, und wobei die Aufbewahrungskammer mit einer Luftzufuhrvorrichtung (24) in Verbindung steht;
wobei das Luftreinigungssystem mit einem Gebläse (12), einer Luftreinigungsvorrichtung (13) und Luftauslässen (10) versehen ist; und
wobei Außenluft des Gehäusekörpers aus Lufteinlässen (14) eingeleitet wird, wobei ein Luftstromkanal (15) in einer Luftausströmrichtung der Luftreinigungsvorrichtung in dem Gehäusekörper ein Reinluftbereich ist, und wobei die Atemfunktionsbaugruppe und die Luftzufuhrvorrichtung jeweils Reinluft ansaugen.

2. Heimbeatmungsgerät nach Anspruch 1, **dadurch gekennzeichnet, dass** der Gehäusekörper (1) ferner mit einem Luftschlauchreinigungs-Verbindungsabschnitt (18) versehen ist, und dass der Luftschlauchreinigungs-Verbindungsabschnitt mit dem Luftstromkanal (15) in Verbindung steht und mit einer beweglichen Abdeckung (5) des Verbindungsabschnitts versehen ist.

3. Heimbeatmungsgerät nach Anspruch 1, **dadurch gekennzeichnet, dass** der Gehäusekörper (1) ferner mit einem Luftschlauchreinigungs-Verbindungsabschnitt (18) versehen ist, wobei der Luftschlauchreinigungs-Verbindungsabschnitt mit der Luftzufuhrvorrichtung (24) in Verbindung steht, wobei die durch die Luftzufuhrvorrichtung (24) angesaugte Reinluft jeweils zu der Aufbewahrungskammer (11) und dem Luftschlauchreinigungs-Verbindungsabschnitt gefördert wird, und wobei der Luftschlauchreinigungs-Verbindungsabschnitt mit einer beweglichen Abdeckung (5) des Verbindungsabschnitts versehen ist.

4. Heimbeatmungsgerät nach Anspruch 1 oder 3, **dadurch gekennzeichnet, dass** ein Ozongenerator (16) in einer Luftausströmrichtung der Luftzufuhrvorrichtung (24) angeordnet ist.

5. Heimbeatmungsgerät nach Anspruch 1 oder 3, **dadurch gekennzeichnet, dass** die Luftzufuhrvorrichtung (24) eine Heizstruktur aufweist.

6. Heimbeatmungsgerät nach Anspruch 1, **dadurch gekennzeichnet, dass** die Atemfunktionsbaugruppe eine Luftzufuhr-Antriebsvorrichtung (20), einen Luftwegkanal, einen Beatmungsschlauchanschluss (9) und eine Befeuchtungsbox (4) umfasst.

7. Heimbeatmungsgerät nach Anspruch 6, **dadurch gekennzeichnet, dass** die Befeuchtungsbox (4) abnehmbar mit dem Gehäusekörper (1) verbunden ist.

8. Heimbeatmungsgerät nach Anspruch 1, **dadurch gekennzeichnet, dass** die Aufbewahrungskammer (11) eine fest oder beweglich montierte Aufbewahrungs- und Platzierungskomponente (23) umfasst, wobei die Aufbewahrungs- und Platzierungskomponente eine Aufbewahrungsplatte oder ein durch Gitter/Roste gebildeter Aufbewahrungskorb ist, wobei die Aufbewahrungsöffnung durch eine Öffnungs- oder Schließtür (3) oder eine bewegliche Abdeckung (29) abgedichtet ist, wobei eine Abdichtungsstruktur zwischen der Aufbewahrungsöffnung und der Öffnungs- oder Schließtür oder der beweglichen Abdeckung angeordnet ist, und wobei die Auslassöffnungen (2) in einer an die Öffnungs- oder Schließtür oder die bewegliche Abdeckung oder die Aufbewahrungskammer angepassten Position ausgebildet sind.

9. Heimbeatmungsgerät nach Anspruch 8, **dadurch gekennzeichnet, dass** die Aufbewahrungsöffnung in einem oberen Ende der Aufbewahrungskammer (11) ausgebildet ist und durch die abnehmbare oder gekoppelte bewegliche Abdeckung (29) verbunden und abgedichtet ist, und dass die Aufbewahrungs- und Platzierungskomponente (23) eine Aufbewahrungsplatte ist.

10. Heimbeatmungsgerät nach Anspruch 8, **dadurch gekennzeichnet, dass** die Aufbewahrungsöffnung in einem Seitenabschnitt der Aufbewahrungskammer (11) ausgebildet ist und durch die gekoppelte Öffnungs- oder Schließtür (3) abgedichtet ist, und dass die Aufbewahrungs- und Platzierungskomponente (23) eine Aufbewahrungsplatte ist.

11. Heimbeatmungsgerät nach Anspruch 8, **dadurch gekennzeichnet, dass** die Aufbewahrungsöffnung in einem Seitenabschnitt der Aufbewahrungskammer (11) ausgebildet ist, wobei die Aufbewahrungs- und Platzierungskomponente (23) ein Aufbewahrungskorb ist, wobei der Aufbewahrungskorb und die Öffnungs- oder Schließtür (3) eine Zugstruktur bilden, um beweglich mit der Aufbewahrungskammer verbunden zu sein, und wobei die Aufbewahrungsöffnung durch die Öffnungs- oder Schließtür abgedichtet ist.

12. Heimbeatmungsgerät nach Anspruch 1, **dadurch gekennzeichnet, dass** die Luftreinigungsvorrichtung (13) aus einem oder mindestens zwei von einem Aktivkohlefilter, einem Kartuschenfilter, einem Anionenreiniger, einem Fotokatalysatorreiniger und einem Plasmareiniger zusammengesetzt ist.

13. Heimbeatmungsgerät nach Anspruch 12, **dadurch gekennzeichnet, dass** der Aktivkohlefilter und der Kartuschenfilter eine fassförmige Struktur oder eine plattenförmige Struktur aufweisen.

14. Heimbeatmungsgerät nach Anspruch 13, **dadurch gekennzeichnet, dass** der Aktivkohlefilter und/oder der Kartuschenfilter mit fassförmiger oder plattenförmiger Struktur durch eine Einbettungsstruktur mit dem Gehäusekörper (1) verbunden sind, und dass eine bewegliche Platte abnehmbar mit einem dem Aktivkohlefilter und/oder dem Kartuschenfilter entsprechenden Abschnitt des Gehäusekörpers (1) verbunden oder daran gekoppelt ist.

15. Heimbeatmungsgerät nach Anspruch 13, **dadurch gekennzeichnet, dass** der Aktivkohlefilter und/oder der Kartuschenfilter mit plattenförmiger Struktur durch eine schubladenartige Zugstruktur konstruiert ist.

16. Heimbeatmungsgerät nach einem der Ansprüche 12, 13, 14 und 15, **dadurch gekennzeichnet, dass** der Kartuschenfilter aus einem oder mindestens zwei von einem Primärfilter, einem Filter mittlerer Effizienz und einem Hocheffizienzfilter zusammengesetzt ist.

17. Heimbeatmungsgerät nach einem der Ansprüche 1, 3, 8, 9, 10 und 11, **dadurch gekennzeichnet, dass** die Aufbewahrungskammer (11) mit mindestens einem Stützelement und/oder einer Sterilisations- und Desinfektionsvorrichtung versehen ist, und dass die Stützelemente Zubehörteile/Komponenten der Atemfunktionsbaugruppe durch mindestens einen Kontaktpunkt oder eine Kontaktfläche tragen, aufhängen und fixieren.

18. Heimbeatmungsgerät nach Anspruch 17, **dadurch gekennzeichnet, dass** die Sterilisations- und Desinfektionsvorrichtung aus einer oder einer Kombination aus mindestens zwei von einer UV-Ultraviolett-Sterilisations- und Desinfektionsstruktur, einer Plasma-Sterilisations- und Desinfektionsstruktur, einer Fotokatalysator-Sterilisations- und Desinfektionsstruktur und einer Ozon-Sterilisations- und Desinfektionsstruktur zusammengesetzt ist.

19. Heimbeatmungsgerät nach Anspruch 1, **dadurch gekennzeichnet, dass** die Anzeigesteuerkomponente eine Reinaufbewahrungs-Anzeigesteuerkomponente (8), einen Atemfunktions-Anzeigebildschirm (7) und eine Atemfunktions-Steuertaste (6) umfasst.

20. Heimbeatmungsgerät nach einem der Ansprüche 1, 2, 3, 7 und 14, **dadurch gekennzeichnet, dass** der Gehäusekörper (1) mit beweglichen Rädern versehen ist.

21. Heimbeatmungsgerät, umfassend einen Gehäusekörper (1) mit einer Atemfunktionsbaugruppe, einer Aufbewahrungsvorrichtung, einem Luftreinigungssystem und einer Anzeigesteuerkomponente, wobei
die Aufbewahrungsvorrichtung mit einer Aufbewahrungskammer (11), Auslassöffnungen (2) und einer Aufbewahrungsöffnung, die geöffnet oder geschlossen werden kann, versehen ist, wobei die Aufbewahrungskammer mit einer Luftzufuhrvorrichtung (24) in Verbindung steht, und wobei die Luftzufuhrvorrichtung Außenluft des Gehäusekörpers ansaugt;
wobei das Luftreinigungssystem mit einem Gebläse (12), einer Luftreinigungsvorrichtung (13) und Luftauslässen (10) versehen ist; und
wobei die Außenluft des Gehäusekörpers aus Lufteinlässen (14) eingeleitet wird, wobei ein Luftstromkanal (15) in einer Luftausströmrichtung der Luftreinigungsvorrichtung in dem Gehäusekörper ein Reinluftbereich ist, und wobei die Atemfunktionsbaugruppe Reinluft ansaugt.

## Revendications

1. Ventilateur domestique comprenant un corps (1) avec un ensemble de fonction respiratoire, un dispositif de stockage, un système de purification de l'air et un composant de commande d'affichage, dans lequel
le dispositif de stockage est pourvu d'une chambre de stockage (11), de ports de décharge (2) et d'une ouverture de stockage pouvant s'ouvrir ou se fermer et la chambre de stockage communique avec un dispositif d'alimentation en air (24) ;
le système de purification de l'air est pourvu d'une soufflante (12), d'un dispositif de purification de l'air (13) et de sorties d'air (10) ; et
l'air extérieur du corps est introduit à partir d'entrées d'air (14), un canal d'écoulement d'air (15) dans une direction de sortie d'air du dispositif de purification de l'air dans le corps est une région d'air propre et l'ensemble de fonction respiratoire et le dispositif d'alimentation en air aspirent de l'air propre respectivement.

2. Ventilateur domestique selon la revendication 1, **caractérisé en ce que** le corps (1) est en outre pourvu d'une partie de raccordement de nettoyage de tube d'air (18) et la partie de raccordement de nettoyage de tube d'air communique avec le canal d'écoulement d'air (15) et est pourvue d'un couvercle mobile de partie de raccordement (5).

3. Ventilateur domestique selon la revendication 1, **caractérisé en ce que** le corps (1) est en outre pourvu d'une partie de raccordement de nettoyage de tube d'air (18), la partie de raccordement de nettoyage de tube d'air communique avec le dispositif d'alimentation en air (24), l'air propre aspiré par le dispositif d'alimentation en air (24) est acheminé respectivement vers la chambre de stockage (11) et la partie de raccordement de nettoyage de tube d'air et la partie de raccordement de nettoyage de tube d'air est pourvue d'un couvercle mobile de partie de raccordement (5).

4. Ventilateur domestique selon la revendication 1 ou 3, **caractérisé en ce qu'**un générateur d'ozone (16) est agencé dans une direction de sortie d'air du dispositif d'alimentation en air (24).

5. Ventilateur domestique selon la revendication 1 ou 3, **caractérisé en ce que** le dispositif d'alimentation en air (24) présente une structure de chauffage.

6. Ventilateur domestique selon la revendication 1, **caractérisé en ce que** l'ensemble de fonction respiratoire comprend un dispositif d'entraînement d'alimentation en air (20), un canal de passage d'air, un raccord de tube respiratoire (9) et une boîte d'humidification (4).

7. Ventilateur domestique selon la revendication 6, **caractérisé en ce que** la boîte d'humidification (4) est reliée de manière détachable au corps (1).

8. Ventilateur domestique selon la revendication 1, **caractérisé en ce que** la chambre de stockage (11) comprend un composant de stockage et d'installation (23) monté de manière fixe ou mobile, le composant de stockage et d'installation est un plateau de stockage ou un panier de stockage formé par des treillis/grilles, l'ouverture de stockage est obstruée par une porte d'ouverture ou de fermeture (3) ou un couvercle mobile (29), une structure d'étanchéité est agencée entre l'ouverture de stockage et la porte d'ouverture ou de fermeture ou le couvercle mobile et les ports de décharge (2) sont formés dans une position pouvant s'adapter à la porte d'ouverture ou de fermeture ou au couvercle mobile ou à la chambre de stockage.

9. Ventilateur domestique selon la revendication 8, **caractérisé en ce que** l'ouverture de stockage est formée dans une extrémité supérieure de la chambre de stockage (11) et est reliée et obstruée par le couvercle mobile (29) détachable ou couplé et le composant de stockage et d'installation (23) est un plateau de stockage.

10. Ventilateur domestique selon la revendication 8, **caractérisé en ce que** l'ouverture de stockage est formée dans une partie latérale de la chambre de stockage (11) et obstruée par la porte d'ouverture ou de fermeture couplée (3) et le composant de stockage et d'installation (23) est un plateau de stockage.

11. Ventilateur domestique selon la revendication 8, **caractérisé en ce que** l'ouverture de stockage est formée dans une partie latérale de la chambre de stockage (11), le composant de stockage et d'installation (23) est un panier de stockage, le panier de stockage et la porte d'ouverture ou de fermeture (3) forment une structure tirable de façon à être reliée de façon mobile à la chambre de stockage et l'ouverture de stockage est obstruée par la porte d'ouverture ou de fermeture.

12. Ventilateur domestique selon la revendication 1, **caractérisé en ce que** le dispositif de purification de l'air (13) est composé d'un seul ou d'au moins deux parmi un filtre à charbon actif, un filtre à cartouche, un purificateur anionique, un purificateur à photocatalyseur et un purificateur à plasma.

13. Ventilateur domestique selon la revendication 12, **caractérisé en ce que** le filtre à charbon actif et le filtre à cartouche présentent une structure en forme de fût ou une structure en forme de plaque.

14. Ventilateur domestique selon la revendication 13, **caractérisé en ce que** le filtre à charbon actif et/ou le filtre à cartouche de la structure en forme de fût ou en forme de plaque est relié au corps (1) par une structure d'encastrement et une plaque mobile est reliée ou couplée de manière détachable à une partie du corps (1) correspondant au filtre à charbon actif et/ou au filtre à cartouche.

15. Ventilateur domestique selon la revendication 13, **caractérisé en ce que** le filtre à charbon actif et/ou le filtre à cartouche présentant une structure en forme de plaque est construit comme une structure tirable de type tiroir.

16. Ventilateur domestique selon l'une quelconque des revendications 12, 13, 14 et 15, **caractérisé en ce que** le filtre à cartouche est composé d'un seul ou d'au moins deux parmi un filtre primaire, un filtre à efficacité moyenne et un filtre à efficacité élevée.

17. Ventilateur domestique selon l'une quelconque des revendications 1, 3, 8, 9, 10 et 11, **caractérisé en ce que** la chambre de stockage (11) est pourvue d'au moins un élément de support et/ou d'un dispositif de stérilisation et de désinfection et les éléments de support portent, suspendent et fixent des accessoires/composants de l'ensemble de fonction respiratoire à travers au moins un point de contact ou une surface de contact.

18. Ventilateur domestique selon la revendication 17, **caractérisé en ce que** le dispositif de stérilisation et de désinfection est composé d'une seule ou d'une combinaison d'au moins deux parmi une structure de stérilisation et de désinfection aux ultraviolets UV, une structure de stérilisation et de désinfection au plasma, une structure de stérilisation et de désinfection à photocatalyseur et une structure de stérilisation et de désinfection à l'ozone.

19. Ventilateur domestique selon la revendication 1, **caractérisé en ce que** le composant de commande d'affichage comprend un composant de commande d'affichage de stockage propre (8), un écran d'affichage de fonction respiratoire (7) et une touche de commande de fonction respiratoire (6).

20. Ventilateur domestique selon l'une quelconque des revendications 1, 2, 3, 7 et 14, **caractérisé en ce que** le corps (1) est pourvu de roues mobiles.

21. Ventilateur domestique comprenant un corps (1) avec un ensemble de fonction respiratoire, un dispositif de stockage, un système de purification de l'air et un composant de commande d'affichage, dans lequel
le dispositif de stockage est pourvu d'une chambre de stockage (11), de ports de décharge (2) et d'une ouverture de stockage pouvant s'ouvrir ou se fermer, la chambre de stockage communique avec un dispositif d'alimentation en air (24) et le dispositif d'alimentation en air aspire l'air extérieur du corps ;
le système de purification de l'air est pourvu d'une soufflante (12), d'un dispositif de purification de l'air (13) et de sorties d'air (10) ; et
l'air extérieur du corps est introduit à partir d'entrées d'air (14), un canal d'écoulement d'air (15) dans une direction de sortie d'air du dispositif de purification de l'air dans le corps est une région d'air propre et l'ensemble de fonction respiratoire aspire de l'air propre.
